(19) Europäisches Patentamt / European Patent Office / Office européen des brevets

(11) **EP 2 272 954 B1**

(12) **EUROPEAN PATENT SPECIFICATION**

(45) Date of publication and mention
of the grant of the patent:
**03.09.2014 Bulletin 2014/36**

(21) Application number: **09734871.8**

(22) Date of filing: **20.04.2009**

(51) Int Cl.:
*C12N 11/00* (2006.01)     *C12N 11/08* (2006.01)
*C12P 7/62* (2006.01)     *C12P 7/64* (2006.01)
*C12N 9/20* (2006.01)

(86) International application number:
**PCT/JP2009/001801**

(87) International publication number:
**WO 2009/130880 (29.10.2009 Gazette 2009/44)**

(54) **METHOD FOR PRODUCTION OF IMMOBILIZED ENZYME**

VERFAHREN ZUR HERSTELLUNG IMMOBILISIERTER ENZYME

PROCÉDÉ DE FABRICATION D'UNE ENZYME IMMOBILISÉE

(84) Designated Contracting States:
**AT BE BG CH CY CZ DE DK EE ES FI FR GB GR
HR HU IE IS IT LI LT LU LV MC MK MT NL NO PL
PT RO SE SI SK TR**

(30) Priority: **21.04.2008 JP 2008110202**

(43) Date of publication of application:
**12.01.2011 Bulletin 2011/02**

(73) Proprietor: **Kao Corporation
Chuo-ku
Tokyo 103-8210 (JP)**

(72) Inventors:
• **FUKUHARA, Shinpei
Kamisu-shi
Ibaraki 314-0103 (JP)**
• **KASE, Minoru
Kamisu-shi
Ibaraki 314-0103 (JP)**
• **KOMATSU, Toshiteru
Kamisu-shi
Ibaraki 314-0103 (JP)**

(74) Representative: **HOFFMANN EITLE
Patent- und Rechtsanwälte
Arabellastrasse 4
81925 München (DE)**

(56) References cited:
EP-A1- 1 378 568      WO-A1-95/33047
JP-A- H0 339 089      JP-A- 63 279 794
JP-A- 2001 252 090      JP-A- 2004 113 238
US-A- 4 933 284

• Kang: "Characteristics of immobilized lipase-catalyzed hydrolysis of olive oil of high concentration in reverse phase system", , May 1989 (1989-05), pages 1469-1476, XP055068062, Retrieved from the Internet: URL:http://onlinelibrary.wiley.com/store/1 0.1002/bit. 260331114/asset/260331114_ftp.p df? v=1&t=hict69wy&s=4705969790cf28cb57cfa6 873722d9b5582bf318 [retrieved on 2013-06-25]

**Description**

Field of the Invention

The present invention relates to a method of producing an immobilized enzyme.

Background of the Invention

In an esterification reaction using glycerin and a fatty acid as raw materials, in order to use an enzyme efficiently, an immobilized enzyme obtained by immobilizing a lipolytic enzyme on an inorganic or organic carrier is used. As the immobilized enzyme is used in the reaction for a long period of time, the activity is lowered. Therefore, it is necessary to collect the enzyme at the time when the activity is lowered to some degree and exchange the enzyme for a fresh immobilized enzyme.

As means for effectively utilizing the collected used immobilized enzyme, there is known a method involving removing all proteins and the like adsorbed thereon with an alkali and reusing the carrier (Patent Document 1). In addition, there is known a method of subjecting an immobilized lipase, which has been used in a transesterification reaction or an interesterification reaction and has lowered activity, to a moist treatment using a solvent or a solvent and a phospholipid to control the moisture content involved in the reaction, to thereby reactivate a remaining lipase (Patent Document 2).

However, of the above-mentioned conventional techniques, the method of removing the enzyme with an alkali is performed using a limited immobilization carrier and cannot be applied directly to an enzyme immobilized on another immobilization carrier.

Moreover, the method of subjecting an immobilized enzyme having lowered activity to a moist treatment using a solvent or a solvent and a phospholipid is employed not for regeneration of the immobilized enzyme having lowered activity by desorption of part of the lipase but for reactivation of the remaining lipase.

Related Art DocumentPatent Document

Patent Document 1: JP-A-01-187086

Patent Document 2: JP-A-09-56379

EP 1 398 374 A1 discloses a method for regenerating an immobilized enzyme for lipolysis which has been used for lipolysis. WO95/33047 describes a method for reusing a carrier for lipase immobilization. In one of its examples, the desorption of a lipase protein from a carrier is carried out using a desorption liquid in the form of a solution of NaOH in 50 % (w/w) aqueous ethanol.

Summary of the Invention

The present invention provides a method of producing an immobilized lipolytic enzyme, including: mixing an immobilized lipolytic enzyme that has been used in an esterification reaction with n-hexane to wash the immobilized lipolytic enzyme so that a residual oil content in the immobilized lipolytic enzyme is reduced to 50 parts by mass or less based on 100 parts by mass of an immobilization carrier; bringing the immobilized lipolytic enzyme into contact with an alkali solution; collecting the immobilization carrier; and adsorbing a lipolytic enzyme on the immobilization carrier.

Detailed Description of the Invention

The present invention relates to a method of producing an immobilized lipolytic enzyme which has almost the same ability as that of an unused immobilized enzyme by effectively utilizing an immobilization carrier in a used immobilized enzyme which has been used in an esterification reaction.

In the case of production of a diacylglycerol (hereinafter, referred to as "DAG") using an immobilized lipolytic enzyme, the DAG purity in a DAG-containing fat and oil was found to decrease when a regenerated immobilized enzyme produced by the conventional techniques as described above was used. Therefore, the inventors of the present invention have made extensive studies for the cause of the decrease in the DAG purity and have found that the phenomenon is caused by an enzyme having lowered activity remaining in the regenerated immobilized enzyme. That is, even if the lipolytic activity is lowered, the interesterification activity remains, and thus, a triacylglycerol (hereinafter, referred to as "TAG") is produced from 1,3-DAG via interesterification into 1,2-DAG, resulting in decrease of the DAG purity. Then, the inventors have made further studies and have found that the enzyme having lowered activity can be completely desorbed from the immobilization carrier that has been used in the esterification reaction by washing the immobilized enzyme with n-hexane and then treating the enzyme with an alkali solution. The inventors have found that the subsequent adsorption of a fresh lipolytic enzyme on the collected immobilization carrier allows an immobilized lipolytic enzyme of interest to be produced.

According to the present invention, it is possible to produce an immobilized lipolytic enzyme which has almost the same activity as that of an unused immobilized enzyme by effectively utilizing an immobilization carrier in an immobilized enzyme which has been used in an esterification reaction. In addition, when the immobilized lipolytic enzyme produced by the method of the present invention is used, a DAG-containing fat and oil with high DAG purity can be produced.

The immobilized enzyme (used immobilized enzyme) for the production method of the present invention is an immobilized lipolytic enzyme which has been used in an esterification reaction and has lowered lipolytic activity. Examples of the esterification reaction include an esterification reaction between glycerin and a fatty acid or a lower alkyl ester thereof as described below.

Examples of the immobilization carrier in the immobilized enzyme for the production method of the present invention include: inorganic carriers such as celite, diatomaceous earth, kaolinite, silica gel, molecular sieves, porous glass, activated carbon, calcium carbonate, and ceramics; and organic polymers such as ceramic powder, polyvinyl alcohol, polypropylene, chitosan, ion exchange resins, hydrophobic adsorption resins, chelating resins, and synthetic adsorption resins. Of those, ion exchange resins are more preferred.

The ion exchange resins are preferably porous anion exchange resins. Such a porous carrier has a large surface area and therefore can adsorb an enzyme in a larger amount. The resin preferably has a particle size of 100 to 1000 μm and a pore size of 10 to 150 nm.

With respect to the anion exchange resin, Patent Document 1 describes that it is difficult to desorb an enzyme inactivated after use to reuse the carrier because the enzyme is hard to desorb from the carrier due to strong adsorption based on hydrophobicity. On the other hand, in the present invention, it has been found that, when an immobilized enzyme is washed with a solvent and then treated with an alkali solution, the carrier can be reused because the enzyme is easily desorbed even from a carrier on which the enzyme has been adsorbed hydrophobically and a fresh enzyme is adsorbed on the carrier.

In the present invention, examples of the material of the anion exchange resin include a phenol-formaldehyde-based resin, a polystyrene-based resin, an acrylamide-based resin, and a divinylbenzene-based resin. Of those, a phenol-formaldehyde-based resin (for example, Duolite A-568 manufactured by Rohm and Hass Co., Ltd.) is more preferred from the standpoint that the effect of the present invention can be attained sufficiently.

In the present invention, an example of the lipolytic enzyme includes a lipase. The type of the lipase maybe arbitrarily selected, and a 1,3-position selective lipase is preferred in terms of production of a DAG-containing fat and oil with high DAG purity.

Commercially available lipases derived from microorganisms as well as lipases derived from animals and plants can be used. Examples of the lipases derived from microorganisms include those derived from the microorganisms belonging to the genera *Rhizopus, Aspergillus, Mucor, Rhizomucor, Pseudomonas, Geotrichum, Penicillium,* and *Candida.*

Examples of the washing method include batch mixing and continuous contact, and in view of handling, the batch mixing is preferred. After washing, the solvent is preferably removed from the immobilized enzyme by a method such as filtration or distillation under reduced pressure.

The washing is performed so that the residual oil content in the immobilized lipolytic enzyme after washing may be reduced to 50 parts by mass (hereinafter, simply referred to as "parts") or less based on 100 parts of the immobilization carrier in view of decreasing the amount of oil adsorbed after washing and reducing saponification between the oil and alkali in an alkali treatment step to promote good handling. The residual oil content in the immobilized lipolytic enzyme after washing is preferably 1 to 50 parts from a similar standpoint, and more preferably 10 to 40 parts from a similar standpoint and treatment cost. The washing procedure may be performed only once, or may be performed several times.

The residual oil content is calculated by measuring the mass of remaining oil based on the mass of the immobilization carrier and using the following equation (1) as a mass ratio based on 100 parts of the immobilization carrier.

$$\text{Residual oil content} = (a-b)/b \times 100 \quad (a: \text{mass of immobilized enzyme}, \; b: \text{mass of immobilization carrier}) \quad (1)$$

The used immobilized enzyme is washed with a solvent, and then brought into contact with an alkali solution to desorb the enzyme. Examples of the alkali to be used in the alkali solution in the present invention include sodium hydroxide, potassium hydroxide, calcium carbonate, and potassium carbonate. In view of property of removing the used enzyme, sodium hydroxide is preferred.

The method for contact with the alkali solution includes batch mixing and continuous contact. In view of handling, the batch mixing is preferred. The contact method includes still standing, stirring, and shaking.

The temperature of the alkali solution to be used for desorption of the enzyme from the used immobilized enzyme is preferably 0 to 70°C, more preferably 30 to 65°C, and even more preferably 40 to 60°C in view of effectively exerting the activity of the immobilized enzyme produced and preventing lowering of the DAG purity. Note that the term "DAG purity" as used herein refers to a mass% (hereinafter, simply referred to as "%") of DAG in DAG and TAG and is represented by the expression: $DAG/(TAG+DAG) \times 100$.

Further, the concentration of the alkali solution is preferably 0.25 to 2 N, and more preferably 0.8 to 1.5 N in view of effectively exerting the activity of the immobilized enzyme produced and preventing lowering of the DAG purity.

The contact time with the alkali solution is preferably 2 to 48 hours, and more preferably 20 to 30 hours in view of effectively exerting the activity of the immobilized enzyme produced and preventing lowering of the DAG purity.

After contact with the alkali solution, the alkali is preferably removed by washing with water, a pH treatment, etc.

In the case of immobilizing a fresh lipolytic enzyme, the enzyme may be adsorbed directly on the immobilization carrier, but the immobilization carrier is preferably treated in advance with a fat-soluble fatty acid or a derivative thereof before adsorbing the enzyme in order to obtain an adsorption state which is likely to exhibit high activity. As for the method of bringing the fat-soluble fatty acid or the derivative thereof into contact with the immobilization carrier, the fat-soluble fatty acid or the derivative thereof may be added directly to water or an organic solvent, or they may be once dispersed and dissolved in an organic solvent in order to improve dispersibility and then the solution may be added to the carrier dispersed in water. Examples of the organic solvent include chloroform, hexane, and ethanol. The mass of the fat-soluble fatty acid or the derivative thereof used therein is preferably 1 to 500 parts, and more preferably 10 to 200 parts based on 100 parts of the immobilization carrier. The contact temperature is preferably 0 to 100°C, and more preferably 20 to 60°C, and the contact time is preferably about 5 minutes to 5 hours. The carrier after this treatment is recovered by filtration, and if necessary, may be dried. The drying temperature is preferably from room temperature to 100°C, and drying may be performed under reduced pressure.

Of the fat-soluble fatty acids or the derivatives thereof to be used for treating the carrier in advance, examples of the fat-soluble fatty acids include saturated or unsaturated, linear or branched fatty acids having 4 to 24 carbon atoms, and preferably 8 to 18 carbon atoms, which may have a hydroxyl group. Specific examples thereof include capric acid, lauric acid, myristic acid, oleic acid, linoleic acid, $\alpha$-linolenic acid, ricinolic acid, and isostearic acid. Further, examples of the derivatives of the fat-soluble fatty acids include esters of these fat-soluble fatty acids with monohydric or polyhydric alcohols or sugars, phospholipids, and derivatives obtained by adding ethylene oxide to these esters. Specific examples thereof include methyl esters, ethyl esters, monoglycerides, and diglycerides of the aforementioned fatty acids, ethylene oxide adducts thereof, polyglycerin esters, sorbitan esters, and sucrose esters thereof. These fat-soluble fatty acids and derivatives thereof are preferably liquids at normal temperature (20°C) for the step of immobilizing the enzyme on the carrier. These fat-soluble fatty acids or the derivatives thereof maybe used in combination of two or more kinds thereof, and naturally occurring fatty acids such as rapeseed fatty acids and soybean fatty acids may also be used.

The temperature for immobilizing the enzyme can be determined according to the characteristics of the enzyme, and is preferably 0 to 60°C, and more preferably 5 to 40°C at which the enzyme is not deactivated. The pH of an enzyme solution used in immobilization may fall within a range where the enzyme is not denatured, and can be determined according to the characteristics of the enzyme as in the case with the temperature, but is preferably pH 3 to 9. In order to maintain the pH range, a buffer solution is used, and the examples of the buffer solution include an acetate buffer solution, a phosphate buffer solution, and a Tris-hydrochloric acid buffer solution.

The enzyme concentration in the enzyme solution described above is preferably not higher than the saturated solubility of the enzyme, while being sufficient, in view of immobilization efficiency. Further, it is also possible to use a supernatant obtained by removing insoluble matters from the enzyme solution by centrifugal separation or a solution obtained by purifying the enzyme solution by ultrafiltration as required. The amount of the enzyme to be used is preferably 5 to 1000 parts, and more preferably 10 to 500 parts based on 100 parts of the immobilization carrier.

In view of enhancing the activity of the immobilized enzyme and the cost of the enzyme, the adsorption ratio of enzyme on the immobilization carrier is preferably as high as possible. The adsorption ratio of enzyme is preferably 50% or more, more preferably 80% ormore, even more preferably 92% ormore, and even pre f erably 94 to 99%. Note that the term "adsorption ratio of enzyme" as used herein refers to the ratio of a remaining activity in the enzyme solution after adsorption of the enzyme (part excluding the immobilized enzyme) based on the activity in the enzyme solution before adsorption of the enzyme.

In the present invention, after the lipolytic enzyme is adsorbed and immobilized on the immobilization carrier, the residual moisture content is preferably adjusted by dehydration by contact with a fat-soluble fatty acid, a fatty acid triglyceride, a fatty acid partial glyceride, or the like without drying.

The residual moisture content is adjusted to preferably 1 to 50 parts, and more preferably 1 to 30 parts based on 100 parts of the immobilization carrier in view of storage stability.

Examples of the fat-soluble fatty acid to be used for adjusting the residual moisture content preferably include fatty acids produced from vegetative liquid fat and oil such as rapeseed oil, soybean oil, and sunflower oil and fish oils such as sardine oil, tuna oil, and bonito oil. Note that fatty acids, fatty acid triglycerides, or fatty acid partial glycerides to be used are preferably selected from those to be used as oil phase substrates in an actual esterification reaction using the immobilized enzyme prepared by the method of the present invention.

The amount of the fatty acid, the fatty acid triglyceride, or the fatty acid partial glyceride to be used for adjusting the residual moisture content is preferably 20 to 3000 parts, and more preferably 100 to 1000 parts based on 100 parts of the immobilization carrier from the viewpoints of sufficient contact with the immobilized enzyme, avoiding wastes due to excess use thereof, enhancing the fluidity, and improving the dehydration efficiency.

When the immobilized enzyme obtained by the method of the present invention (hereinafter, referred to as "immobilized enzyme of the present invention") is used to produce DAG, fat and oil containing highly-pure DAG (the ratio of DAG in DAG+TAG is high) can be prepared. The DAG purity in the DAG-containing fat and oil is preferably 50% or more, more preferably 65% or more, even more preferably 80 to 100%, and even more preferably 93 to 98% in view of the physiological function. Meanwhile, the TAG content in the DAG-containing fat and oil is preferably 20% or less, more preferably 10% or less, even more preferably 5% or less, and even more preferably 4% or less.

In addition, when the immobilized enzyme of the present invention is used to produce DAG, DAG-containing fat and oil with almost the same DAG purity as that in the case of production of DAG using an immobilized enzyme obtained by adsorbing an enzyme on an unused immobilization carrier (hereinafter, referred to as "fresh immobilized enzyme") can be obtained. The difference between the DAG purity in the case of using the immobilized enzyme of the present invention and the DAG purity in the case of using the fresh immobilized enzyme, that is, the "difference in DAG purity" of the DAG-containing fat and oil, defined by the following equation (2) is preferably -0.9% or more, more preferably -0.7% or more, and even more preferably -0.5% or more.

$$\text{Difference in DAG purity} = (\text{DAG purity in the case of using the immobilized enzyme of the present invention}) - (\text{DAG purity in the case of using the fresh immobilized enzyme}) \quad (2)$$

Meanwhile, the difference between the TAG content in the case of using the immobilized enzyme of the present invention and the TAG content in the case of using the fresh immobilized enzyme, that is, the "difference in TAG content" of the DAG-containing fat and oil, defined by the following equation (3) is preferably 0.6% or less, and more preferably 0.3% or less.

$$\text{Difference in TAG content} = (\text{TAG content in the case of using the immobilized enzyme of the present invention}) - (\text{TAG content in the case of using the fresh immobilized enzyme}) \quad (3)$$

Examples of the method of producing DAG in the present invention include an esterification reaction between glycerin and a fatty acid or a lower alkyl ester thereof.

Here, preferred examples of the fatty acid or lower alkyl ester thereof used in the esterification reaction include linear or branched, saturated or unsaturated fatty acids having 4 to 22 carbon atoms, and preferably 8 to 18 carbon atoms, such as butyric acid, valeric acid, caproic acid, enanthic acid, caprylic acid, pelargonic acid, capric acid, undecanoic acid, lauric acid, myristic acid, palmitic acid, zoomaric acid, stearic acid, oleic acid, elaidic acid, linoleic acid, linolenic acid, arachidonic acid, gadoleic acid, arachic acid, behenic acid, erucic acid, eicosapentaenoic acid, and docosahexaenoic acid. In addition, examples of the lower alcohol which forms an ester with the fatty acid described above include a lower alcohol having 1 to 6 carbon atoms, such as methanol, ethanol, 1-propanol, 2-propanol, n-butanol, 2-butanol, ort-butanol. The fatty acids or lower alkyl esters thereof may be used in combination of two or more kinds thereof. Moreover, a mixture of the aforementioned fatty acids, e.g., a naturally occurring fatty acid such as soybean fatty acid may also be used.

In this reaction, the reaction molar ratio R of the fatty acid or lower alkyl ester thereof and glycerin [R=fatty acid or lower alkyl ester thereof (mol)/glycerin (mol)] is preferably 1.5 to 3.0, more preferably 1.6 to 2.8, and even more preferably 1.8 to 2.6.

In addition, the reaction temperature of the esterification reaction is not particularly limited and is preferably 20 to 80°C, and more preferably 30 to 70°C in view of the reactivity. Meanwhile, the reaction time is preferably within 10 hours from the viewpoint of industrial productivity.

The DAG-containing fat and oil obtained by the esterification reaction can be processed into a product after undergoing post-treatments. Asthepost-treatments, deacidification (removal of an unreacted fatty acid and a monoacylglycerol produced as a by-product), acid treatment, water washing, and deodorization steps are each preferably performed. The deacidification step is a step involving removing an unreacted fatty acid and a monoacylglycerol produced as a by-product from reaction products by performing distillation of DAG-containing fat and oil obtained by the esterification reaction under reduced pressure. The acid treatment step is a step involving addition of a chelating agent such as citric acid to the deacidified oil, mixing, and if necessary, additional dehydration under reduced pressure. Further, the resultant acid-treated oil may be subjected to a decolorization step by contact with an adsorbent to further improve its color and flavor. The water washing step is a step involving separating the oil and water by adding water to the acid-treated oil and stirring the mixture vigorously. The water washing is preferably repeated several times (e.g., three times) to obtain a water-washed oil. The deodorization step is a step involving performing steam distillation under reduced pressure for the water-washed oil. Examples of the deodorization include batch deodorization, continuous deodorization, and semi-continuous deodorization, and a deodorization treatment may be performed using either a thin-film deodorization device or a tray-type deodorization device singly, or may be performed by combining the deodorization treatment using a thin-film deodorization device and the deodorization treatment using a tray-type deodorization device.

Examples

[Immobilized enzyme activity measurement method]

Four parts of an immobilized enzyme were charged into a four-neck flask equipped with crescent blades, and washed three times with about 20 parts of soybean fatty acid. Thereafter, soybean fatty acid was added, and the whole was stirred at 50°C and 400 rpm for 15 minutes. Subsequently, glycerin was added to initiate the reaction, and the pressure was reduced using a vacuum pump. The esterification reaction was performed at a temperature of 50°C with stirring at 400 rpm and at a vacuum degree of 400 Pa. The soybean fatty acid and glycerin were used at an FA/GLY molar ratio of 2, and the total charge amount was 80 parts. The reaction solution was collected every 30 minutes, and the AV value, moisture content, and glyceride composition were analyzed to continuously determine changes over time in the respective compositions (FA, GLY, MAG, DAG, and TAG). The time when the yield (DAG+TAG) reached 70% and DAG purity (mass of DAG/ (mass of DAG+TAG) $\times$ 100) were calculated from the time-dependent curve.

[Preparation of fresh immobilized enzyme A]

Ten parts of Duolite A-568 (Rohm and Hass) were stirred in 100 parts of a 0.1 N aqueous sodium hydroxide solution for 1 hour. The resultant was filtered, and the carrier was washed with 100 parts of distilled water and pH-equilibrated once with 100 parts of a 500 mM acetate buffer (pH 6) and twice with 100 parts of a 50 mM acetate buffer (pH 6). The mixture was filtered, and ethanol substitution was performed with 40 parts of ethanol. After filtration, a mixture of 10 parts of soybean fatty acid and 40 parts of ethanol was added, and the whole was stirred for 30 minutes. Subsequently, washing was performed with 50 parts of 50 mM acetate buffer (pH 6) four times for 30 minutes each, and the carrier was collected by filtration. Thereafter, the carrier was brought into contact with an enzyme solution obtained by dissolving 10 parts of a lipase (Lilipase A-10FG, Nagase ChemteX Corporation) in 180 parts of a 50 mM acetate buffer (pH 6) for 2 hours to immobilize the enzyme. After immobilization, the immobilized enzyme was collected by filtration and washed with 50 parts of a 50 mM acetate buffer (pH 6) for 30 minutes to remove a non-immobilized enzyme and protein, followed by filtration to collect the immobilized enzyme. The adsorption ratio of enzyme was found to be 98.0%. Subsequently, the immobilized enzyme collected was brought into contact with 40 parts of rapeseed oil for 16 hours, and filtration was performed to collect an oil-treated immobilized enzyme. The activity of a fresh immobilized enzyme A, prepared in accordance with the above-mentioned procedure, was measured by the above-mentioned "Immobilized enzyme activity measurement method" (this method is applied to the following sections), and as a result, the time when the yield reached 70%, the DAG purity, and the TAG content were found to be 64.1 minutes, 95.1%, and 3.4%, respectively. The results are shown in Table 1.

[Preparation of fresh immobilized enzyme B]

Ten parts of Duolite A-568 (Rohm and Hass) were stirred in 100 parts of a 0.1 N aqueous sodium hydroxide solution for 1 hour. Filtration was performed, and the carrier was washed with 100 parts of distilled water and pH-equilibrated once with 100 parts of a 500 mM acetate buffer (pH 6) and twice with 100 parts of a 50 mM acetate buffer (pH 6), followed by filtration to collect the carrier. Thereafter, the carrier was brought into contact with an enzyme solution obtained by dissolving 12 parts of a lipase (Palatase 20000L, Novozymes Japan Ltd.) in 180 parts of a 50 mM acetate buffer (pH 6) for 2 hours to immobilize the enzyme. After immobilization, the immobilized enzyme was collected by filtration and washed with 50 parts of a 50 mM acetate buffer (pH 6) for 30 minutes to remove a non-immobilized enzyme and protein, followed by filtration to collect the immobilized enzyme. The adsorption ratio of enzyme was found to be 99.8%. Subsequently, the immobilized enzyme collected was brought into contact with 40 parts of rapeseed oil for 16 hours, and filtration was performed to collect an oil-treated immobilized enzyme. The activity of a fresh immobilized enzyme B, prepared in accordance with the above-mentioned procedure, was measured, and as a result, the time when the yield reached 70%, the DAG purity, and the TAG content were found to be 117.7 minutes, 97.0%, and 2.1%, respectively. The results are shown in Table 2.

[Preparation of used immobilized enzyme A]

The fresh immobilized enzyme A was subjected to a procedure corresponding to a 1000-hour esterification reaction at 50°C, to thereby prepare a used immobilized enzyme A with lowered activity and a remaining activity. The residual oil content was found to be 150 parts based on 100 parts of the immobilization carrier. The activity of the used immobilized enzyme A was measured, and as a result, the time when the yield reached 70%, the DAG purity, and the TAG content were found to be 220 minutes, 92.9%, and 5.0%, respectively.

[Preparation of used immobilized enzyme B]

The fresh immobilized enzyme B was subjected to a procedure corresponding to a 1000-hour esterification reaction at 50°C, to thereby prepare a used immobilized enzyme B with lowered activity and a remaining activity. The residual oil content was found to be 85.3 parts based on 100 parts of the immobilization carrier. The activity of the used immobilized enzyme B was measured, and as a result, the time when the yield reached 70%, the DAG purity, and the TAG content were found to be 290 minutes, 93.9%, and 4.4%, respectively.

[Measurement of residual oil content]

An immobilized enzyme ("a" parts) was washed with 10-fold mass of hexane and acetone alternately three times each, and the whole was left to stand at 70°C for 15 hours to eliminate the solvent. The mass of only the immobilization carrier was determined (b), and the residual oil content was calculated by the following equation as a mass ratio based on 100 parts of the immobilization carrier.

$$\text{Residual oil content} = (a-b)/b \times 100 \quad (a: \text{mass of immobilized enzyme, } b: \text{mass of immobilization carrier})$$

[Measurement of adsorption ratio of enzyme]

The enzymatic activity in the enzyme solution before and after adsorption in the enzyme immobilization procedure was measured using Lipase Kit S (manufactured by Dainippon Pharmaceutical Co., Ltd.) after performing a reaction at 37 °C for 15 minutes, and the adsorption ratio of enzyme was calculated by the following equation.

$$\text{Adsorption ratio of enzyme } [\%] = (\text{Activity before adsorption} - \text{activity after adsorption})/\text{Activity before adsorption} \times 100$$

Example 1

To 10 parts of the used immobilized enzyme A (dry basis) were added 85 parts of n-hexane, and the mixture was stirred for 30 minutes. The residual oil content after washing was found to be 36.9 parts based on 100 parts of the immobilization carrier. The hexane was distilled off under reduced pressure, and the immobilized enzyme was brought into contact with 100 parts of a 1 N aqueous sodium hydroxide solution at 50°C for 24 hours to desorb the residual enzyme. The aqueous sodium hydroxide solution was removed, and the carrier was washed with 100 parts of distilled water and neutralized with 4 parts of a 10% aqueous acetic acid solution and 100 parts of distilled water. Thereafter, the carrier was pH-equilibrated once with 100 parts of a 500 mM acetate buffer (pH 6) and twice with 100 parts of a 50 mM acetate buffer (pH 6). Filtration was performed, and ethanol substitution was performed with 40 parts of ethanol. Filtration was performed, and a mixture of 10 parts of soybean fatty acid and 40 parts of ethanol was added, followed by stirring for 30 minutes. Subsequently, washing was performed four times with 50 parts of a 50 mM acetate buffer (pH 6) for 30 minutes each, and the carrier was collected by filtration. Thereafter, the carrier was brought into contact with an enzyme solution obtained by dissolving 10 parts of a lipase (Lilipase A-10FG, Nagase Chemtex Corporation) in 180 parts of a 50 mM acetate buffer (pH 6) for 2 hours to immobilize the enzyme. After immobilization, the immobilized enzyme was collected by filtration and washed with 50 parts of a 50 mM acetate buffer (pH 6) for 30 minutes to remove a non-immobilized enzyme and protein, followed by filtration to collect the immobilized enzyme. The adsorption ratio of enzyme was found to be 97.7%. Subsequently, the immobilized enzyme collected was brought into contact with 40 parts of rapeseed oil for 16 hours, and filtration was performed to collect an oil-treated immobilized enzyme.

The activity of the immobilized enzyme produced in accordance with the above-mentioned procedure was measured, and as a result, the time when the yield reached 70%, the DAG purity, the difference in DAG purity, the TAG content, and the difference in TAG content were found to be 64.3 minutes, 95.0%, -0.1%, 3.5%, and 0.1%, respectively. The results are shown in Table 1.

Example 2

An immobilized enzyme was prepared in the same way as in Example 1 except that the contact time with the aqueous sodium hydroxide solution was 2 hours. The adsorption ratio of enzyme was found to be 95.5%. The activity of the resultant immobilized enzyme was measured, and as a result, the time when the yield reached 70%, the DAG purity, the difference in DAG purity, the TAG content, and the difference in TAG content were found to be 66.2 minutes, 94.7%, -0.4%, 3.8%, and 0.3%, respectively. The results are shown in Table 1.

Example 3

To 10 parts of the used immobilized enzyme A (dry basis) were added 85 parts of n-hexane, and the mixture was stirred for 30 minutes. The hexane was distilled off under reduced pressure. The procedure was performed twice, and a product having a residual oil content of 6.7 parts based on 100 parts of the immobilization carrier was used to prepare an immobilized enzyme in the same way as in Example 1. The adsorption ratio of enzyme was found to be 96.7%. The activity of the resultant immobilized enzyme was measured, and as a result, the time when the yield reached 70%, the DAG purity, the difference in DAG purity, the TAG content, and the difference in TAG content were found to be 65.2 minutes, 96.7%, 1.6%, 2.3%, and -1.1%, respectively. The results are shown in Table 1.

Example 4

To 10 parts of the used immobilized enzyme A (dry basis) were added 85 parts of n-hexane, and the mixture was stirred for 30 minutes. The hexane was distilled off under reduced pressure. The procedure was performed three times, and a product having a residual oil content of 2.4 parts based on 100 parts of the immobilization carrier was used to prepare an immobilized enzyme in the same way as in Example 1. The adsorption ratio of enzyme was found to be 98.6%. The activity of the resultant immobilized enzyme was measured, and as a result, the time when the yield reached 70%, the DAG purity, the difference in DAG purity, the TAG content, and the difference in TAG content were found to be 66.8 minutes, 95.8%, 0.7%, 2.9%, and -0.5%, respectively. The results are shown in Table 1.

Example 5

To 10 parts of the used immobilized enzyme B (dry basis) were added 85 parts of n-hexane, and the mixture was stirred for 30 minutes. The residual oil content after washing was found to be 8.7 parts based on 100 parts of the immobilization carrier. The hexane was distilled off under reduced pressure, and the immobilized enzyme was brought into contact with 100 parts of a 1 N aqueous sodium hydroxide solution at 50°C for 24 hours to desorb the residual enzyme. The aqueous sodium hydroxide solution was removed, and the carrier was washed with 100 parts of distilled water and neutralized with 4 parts of a 10% aqueous acetic acid solution and 100 parts of distilled water. Thereafter, the carrier was pH-equilibrated once with 100 parts of a 500 mM acetate buffer (pH 6) and twice with 100 parts of a 50 mM acetate buffer (pH 6). The carrier was collected by filtration. Thereafter, the carrier was brought into contact with an enzyme solution obtained by dissolving 12 parts of a lipase (Palatase 20000 L, Novozymes Japan Ltd.) in 180 parts of a 50 mM acetate buffer (pH 6) for 2 hours to immobilize the enzyme. After immobilization, the immobilized enzyme was collected by filtration and washed with 50 parts of a 50 mM acetate buffer (pH 6) for 30 minutes to remove a non-immobilized enzyme and protein, followed by filtration to collect the immobilized enzyme. The adsorption ratio of enzyme was found to be 99.1%. Subsequently, the immobilized enzyme collected was brought into contact with 40 parts of rapeseed oil for 16 hours, and filtration was performed to collect an oil-treated immobilized enzyme.

The activity of the resultant immobilized enzyme was measured, and as a result, the time when the yield reached 70%, the DAG purity, the difference in DAG purity, the TAG content, and the difference in TAG content were found to be 86.8 minutes, 97.1%, 0.1%, 2.1%, and 0.0%, respectively. The results are shown in Table 2.

Comparative Example 1

The above-mentioned used immobilized enzyme A was brought into contact with 100 parts of a 1 N aqueous sodium hydroxide solution at 50°C for 24 hours without washing with hexane to desorb the remaining enzyme. However, it was impossible to filter the liquid due to its low fluidity, resulting in failure of the preparation of an immobilized enzyme.

Comparative Example 2

To 10 parts of the above-mentioned used immobilized enzyme A (dry basis) were added 30 parts of n-hexane to wash the immobilized enzyme so that the residual oil content was 100 parts based on 100 parts of the immobilization carrier. Thereafter, the immobilized enzyme was brought into contact with 100 parts of a 1 N aqueous sodium hydroxide solution at 50 °C for 24 hours to desorb the remaining enzyme. However, it was impossible to filter the liquid due to its low fluidity, resulting in failure of the preparation of an immobilized enzyme as in Comparative Example 1.

Comparative Example 3

An immobilized enzyme was prepared in the same way as in Example 1 except that ethanol substitution was performed after washing with hexane without desorbing the enzyme by the alkali treatment. The adsorption ratio of enzyme was found to be 91.1%. The activity of the resultant immobilized enzyme was measured, and as a result, the time when the yield reached 70%, the DAG purity, the difference in DAG purity, the TAG content, and the difference in TAG content were found to be 69.5 minutes, 92.6%, -2.5%, 5.2%, and 1.8%, respectively. The results are shown in Table 1.

Comparative Example 4

An immobilized enzyme was prepared in the same way as in Example 5 except that enzyme immobilization was performed after washing with hexane without desorbing the enzyme by the alkali treatment. The adsorption ratio of enzyme was found to be 93.8%. The activity of the resultant immobilized enzyme was measured, and as a result, the time when the yield reached 70%, the DAG purity, the difference in DAG purity, the TAG content, and the difference in TAG content were found to be 103.8 minutes, 96.0%, -1.0%, 2.8%, and 0.7%, respectively. The results are shown in Table 2.

[Table 1]

| | Fresh immobilized enzyme A | Example | | | | Comparative Example | | |
|---|---|---|---|---|---|---|---|---|
| | | 1 | 2 | 3 | 4 | 1 | 2 | 3 |
| Residual oil content (parts based on 100 parts of immobilization carrier) | 0.0 | 36.9 | 36.9 | 6.7 | 2.4 | 150 | 100 | 36.9 |
| Adsorption ratio of enzyme (%) | 98.0 | 97.7 | 95.5 | 96.7 | 98.6 | - | - | 91.1 |
| Time when yield reached 70% (min) | 64.1 | 64.3 | 66.2 | 65.2 | 66.8 | - | - | 69.5 |
| DAG purity (%) | 95.1 | 95.0 | 94.7 | 96.7 | 95.8 | - | - | 92.6 |

(continued)

| | Fresh immobilized enzyme A | Example | | | | Comparative Example | | |
|---|---|---|---|---|---|---|---|---|
| | | 1 | 2 | 3 | 4 | 1 | 2 | 3 |
| Difference in DAG purity (%) | - | -0.1 | -0.4 | 1.6 | 0.7 | - | - | -2.5 |
| TAG content (%) | 3.4 | 3.5 | 3.8 | 2.3 | 2.9 | - | - | 5.2 |
| Difference in TAG content (%) | - | 0.1 | 0.3 | -1.1 | -0.5 | - | - | 1.8 |

[Table 2]

| | Fresh immobilized enzyme B | Example | Comparative Example |
|---|---|---|---|
| | | 5 | 4 |
| Residual oil content (parts based on 100 parts of immobilization carrier) | 0.0 | 8.7 | 8.7 |
| Adsorption ratio of enzyme (%) | 99.8 | 99.1 | 93.8 |
| Time when yield reached 70% (min) | 117.7 | 86.8 | 103.8 |
| DAG purity (%) | 97.0 | 97.1 | 96.0 |
| Difference in DAG purity (%) | - | 0.1 | -1.0 |
| TAG content (%) | 2.1 | 2.1 | 2.8 |
| Difference in TAG content (%) | - | 0.0 | 0.7 |

The results of Tables 1 and 2 reveal that: an immobilized lipolytic enzyme having an activity equal to that of a newly produced immobilized enzyme can be produced by washing a used immobilized lipolytic enzyme with a solvent to reduce the residual oil content in the immobilized lipolytic enzyme to 50 parts or less based on 100 parts of an immobilization carrier, performing an alkali treatment, and then adsorbing the lipolytic enzyme on the carrier; and when the thus-produced immobilized enzyme is used in an esterification reaction, DAG-containing fat and oil with high DAG purity can be produced.

**Claims**

1.    A method of producing an immobilized lipolytic enzyme, comprising:

mixing an immobilized lipolytic enzyme that has been used in an esterification reaction with n-hexane to wash the immobilized lipolytic enzyme so that a residual oil content in the immobilized lipolytic enzyme is reduced to 50 parts by mass or less based on 100 parts by mass of an immobilization carrier;
bringing the immobilized lipolytic enzyme into contact with an alkali solution;
collecting the immobilization carrier; and
adsorbing a lipolytic enzyme on the immobilization carrier.

2.    The method of producing an immobilized lipolytic enzyme according to claim 1, wherein the alkali solution has a temperature of 0 to 70°C and a concentration of 0.25 to 2 N, and a contact time with the alkali solution is 2 to 48 hours.

3.    The method of producing an immobilized lipolytic enzyme according to claim 1 or 2, wherein the lipolytic enzyme comprises a 1,3-position selective lipase.

4.    The method of producing an immobilized lipolytic enzyme according to claim 1 or 2, wherein the immobilization carrier comprises an anion exchange resin.

**Patentansprüche**

1.  Verfahren zur Herstellung von immobilisiertem lipolytischem Enzym, umfassend:

    Mischen von immobilisiertem lipolytischem Enzym, das in einer Veresterungsreaktion verwendet worden war, mit n-Hexan, um das immobilisierte lipolytische Enzym zu waschen, so dass der Gehalt an Ölrückständen in dem immobilisierten lipolytischen Enzym auf 50 Masseteile oder weniger in bezug auf 100 Masseteile eines Immobilisierungsträgers reduziert wird;
    In-Kontakt-Bringen des immobilisierten lipolytischen Enzyms mit einer Alkalilösung;
    Sammeln des Immobilisierungsträgers; und
    Adsorbieren eines lipolytischen Enzyms auf dem Immobilisierungsträgers.

2.  Verfahren zur Herstellung eines immobilisierten lipolytischen Enzyms gemäss Anspruch 1, wobei die Alkalilösung eine Temperatur von 0 bis 70°C und eine Konzentration von 0,25 bis 2 N aufweist und eine Kontaktzeit mit der Alkalilösung von 2 bis 48 Stunden beträgt.

3.  Verfahren zur Herstellung eines immobilisierten lipolytischen Enzyms gemäss Anspruch 1 oder 2, wobei das lipolytische Enzym eine für die 1,3-Position selektive Lipase umfasst.

4.  Verfahren zur Herstellung eines immobilisierten lipolytischen Enzyms gemäss Anspruch 1 oder 2, wobei der Immobilisierungsträger ein Anionenaustauschharz umfasst.

**Revendications**

1.  Procédé de production d'une enzyme lipolytique immobilisée, comprenant le fait :

    de mélanger une enzyme lipolytique immobilisée qui a été utilisée dans une réaction d'estérification avec du n-hexane pour laver l'enzyme lipolytique immobilisée de sorte que la teneur en huile résiduelle dans l'enzyme lipolytique immobilisée soit réduite à une valeur inférieure ou égale à 50 parties en masse sur la base de 100 parties en masse d'un support d'immobilisation ;
    de mettre l'enzyme lipolytique immobilisée en contact avec une solution alcaline ;
    de collecter le support d'immobilisation ; et
    d'adsorber une enzyme lipolytique sur le support d'immobilisation.

2.  Procédé de production d'une enzyme lipolytique immobilisée selon la revendication 1, dans lequel la solution alcaline a une température allant de 0 à 70°C et une concentration allant de 0,25 à 2 N, et un temps de contact avec la solution alcaline est de 2 à 48 heures.

3.  Procédé de production d'une enzyme lipolytique immobilisée selon la revendication 1 ou 2, dans lequel l'enzyme lipolytique comprend une lipase sélective des positions 1, 3.

4.  Procédé de production d'une enzyme lipolytique immobilisée selon la revendication 1 ou 2, dans lequel le support d'immobilisation comprend une résine échangeuse d'anions.

**EP 2 272 954 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 1187086 A
- JP 9056379 A
- EP 1398374 A1
- WO 9533047 A